# EUROPEAN PATENT SPECIFICATION

(11) **EP 2 873 388 B1**
(45) Date of publication and mention of the grant of the patent: **04.10.2017**
(21) Application number: 13193514.0
(22) Date of filing: 19.11.2013
(51) Int. Cl.: A61C 1/00

(54) **Soft-tissue laser surgery**
Weichgewebe-Laserchirurgie
Chirurgie laser de tissus mous

(43) Date of publication of application: 20.05.2015
(73) Proprietor: Sirona Dental Systems GmbH, 64625 Bensheim (DE)
(72) Inventor: Sutter, Ralf, 69469 Weinheim (DE); Oehme, Bernd, 55128 Mainz (DE); Jerger, Thomas, 72116 Mössingen (DE); Zanata, Francesco, 31050 Ponzano Veneto, TV (IT)
(74) Representative: Sommer, Peter

(56) References cited:
- EP-A2- 1 281 370
- WO-A1-03/059305
- WO-A2-96/36396
- WO-A2-2009/003014
- DE-A1-102012 007 300

## Description

### Field of the invention

The present invention relates to the field of devices for the treatment of the human or animal body by surgery in particular in dentistry.

It has become customary in dentistry to cut soft tissue, in particular gum or gingiva, or to disinfect pockets in the gingiva that exist at the neck of teeth with the help of lasers (pereodontology). The same applies to a disinfection of the channel in the root of teeth (endodontic treatment). Often, and in particular in other areas of surgery these methods are summarized as soft-tissue laser surgery. In general, surgery requires higher powers of laser light than pereodontology.

The light of these lasers is coupled into optical fibres. It reaches the distal end of the optical fibre. This end of the optical fibre is brought in contact with the parts of the body that are to be treated.

### Description of related art

Prior art concepts of cutting soft tissue, particularly in dentistry, rely on the use of red or infrared diode lasers. The typical wavelengths employed are 810 ± 15 nm, 940 nm or 975 ± 15 nm. These diode lasers, however, must use very high intensities for cutting soft tissue. This leads to excessive thermal heating of the surrounding tissue with corresponding side-effects.

WO 2009/003014 A2 shows headpieces for a wide range of dental treatments, wave lengths and power ranges.

### Brief summary of the invention

It is, therefore, an object of the present invention to provide a device for treatment of the human or animal body by surgery, in particular in den-tistry, with reduced side effects.

This aim is achieved by the invention as claimed in the independent claim. Advantageous embodiments are described in the dependent claim.

We suggest a device for treatment of the human or animal body by surgery with the help of laser light, in particular for dentistry, comprising a laser diode with a wavelength of 445 ± 15 nm, in particular 445 ± 10 nm, for providing the laser light.

The use of wavelengths in the range of 445 nm has the advantage of having a much lower cost per 1 W of light power than other wavelengths. This allows for smaller power supplies like batteries, without compromising performance.

Furthermore, the absorption constant of haemoglobin is two orders of magnitude higher at 445 nm than at e.g. 800 nm. For melanin there is an increase in the absorption constant by a factor of about 3. Therefore, a much lower intensity of the light for cutting soft tissue is required, typically only 1 to 3 W. Combining this with the lower costs per 1 W of light power results in overall largely reduce costs for cutting soft tissue.

The lower required light power has the additional advantage of producing less secondary heat and therefore requiring less cooling of the laser diode. Even the cooling with the ambient atmosphere can be sufficient, whilst prior art lasers for surgical applications have to be cooled typically by a Peltier element.

The lower required light power also has the advantage of almost entirely avoiding thermal damaging of the tissue.

Due to the increased absorption, also the penetration depth of the laser ray is reduced. Thus, there is no additional damage to the tissue in layers below the surface.

The increased absorption efficiency also increases the speed of cutting and therefore decreases the time of treatment.

Furthermore, prior art lasers using red and infrared light had to get in contact with the tissue for cutting. Usually, the end of the optical fibre would be covered by a black coating, e.g. soot, a so-called conditioning process. This black coating would absorb the red or infrared light and, thus, heat the tip of the optical fibre. This heated fibre tip would then be used for cutting the tissue by pushing the hot fibre tip through the tissue.

Due to the increased absorption efficiency of light at 445 nm by the haemoglobin in the tissue, it is now possible to make a transition to non-contact cutting. The tip of the fibre is brought in close vicinity, but not in contact with the tissue to be cut. The light at 445 nm leaves the optical fibre and evaporates the tissue due to the effective absorption.

Non-contact cutting, in turn, exerts less strain on the optical fibre. The optical fibre, therefore, has a longer effective life span.

The use of even shorter wavelengths, for example 405 nm, has the disadvantage that these lasers are very expensive, particularly when looking at the amount of money that has to be spent for 1 W of light power. Also, 405 nm is too close to ultraviolet light and could cause cancer in the affected region or other side effects.

As an additional benefit, natural tissue fluoresces when excited at 445 nm. Therefore, in addition to the excitation light of 445 nm, a fluorescence spot can be seen by the surgeon. Therefore, the surgeon always knows exactly what spot of tissue is currently being treated.

The fluorescence also shows whether tissue is actually being treated.

The wavelength of 445 nm can also be used to cure composites, which are used as dental filling.

Furthermore, due to the low absorption of blue light in water, the area of treatment can be cooled using water, without the functionality of the laser being notably reduced. This is particularly important if a treatment is undergone in close vicinity to bones, because that way the risk of necrosis is reduced.

Prior art devices using infrared light used about 10 W of electrical input power to the laser diode. With a conversion effectiveness of about 50 % of electrical to optical power, this leads to about 5 W of infrared optical power.

This is sufficient to cut the tissue. On the other hand, this means 5 W of additional thermal heating in the device, which requires a corresponding active cooling.

The proposed device has an electrical power supply for the laser diode, wherein the power supplied to the laser diode is below or equal to 3 W of electrical power. With 50 % effectiveness, this leads to 1.5 W of blue laser light, which is sufficient for cutting the tissue.

On the other hand, this leads to only 1.5 W of thermal heating in the device. This amount of thermal heating can be absorbed by the surrounding air and the hand of the operator.

Therefore, passive cooling is sufficient for the claimed device.

The device according to the invention can be used in a pro - cess comprising carrying out a treatment of the human or animal body by surgery or therapy with the help of laser light, in particular for dentistry.

Brief description of the several views of the drawings Other objects and advantages of the present invention may be ascertained from a reading of the specification and appended claims in conjunction with the drawings therein.

For a more complete understanding of the present invention, reference is established to the following description made in connection with accompanying drawings in which:

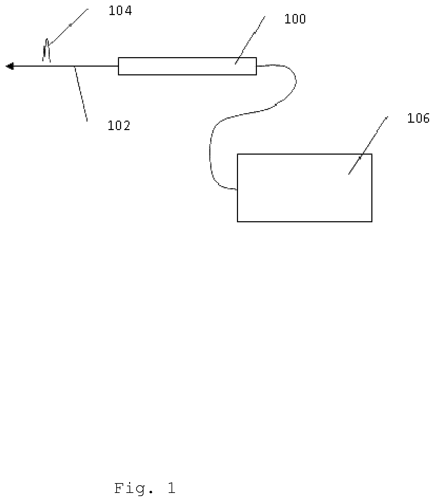

Fig. 1 shows a schematic view of the device for treatment of the human or animal body by surgery with the help of laser light.

### Detailed description of the invention

Fig. 1 shows the device 100 for treatment of the human or animal body by surgery with the help of laser light 102. The laser light 102 is generated by a laser dio-de with a wavelength of 445 ± 20 nm, in particular 445 ± 10 nm (rf. 104). The laser diode is powered by a power supply 106.

While the present inventions have been described and illustrated in conjunction with a number of specific embodiments, those skilled in the art will appreciate that variations and modifications may be made without departing from the principles of the inventions as herein illustrated, as described and claimed. The present inventions may be embodied in other specific forms without departing from their spirit or essential characteristics. The described embodiments are considered in all respects to be illustrative and not restrictive. The scope of the invention is, therefore, indicated by the appended claims, rather than by the foregoing description. All changes which come within the meaning and range of equivalence of the claims are to be embraced within their scope.

### Glossary

### dentistry

Dentistry is the branch of medicine that is involved in the study, diagnosis, prevention, and treatment of diseases, disorders and conditions of the oral cavity, the maxillofacial area and the adjacent and associated structures, and their impact on the human body. (see https://en.wikipedia.org/wiki/Dentistry)

### laser diode

A laser diode is an electrically pumped semiconductor laser in which the active medium is formed by a p-n junction of a semiconductor diode similar to that found in a light-emitting diode. The laser diode is distinct from the optically pumped semiconductor laser, in which, while also semiconductor based, the medium is pumped by a light beam rather than electric current. (see
https://en.wikipedia.org/wiki/Laser diode)

### laser light

A laser is a device that emits light through a process of optical amplification based on the stimulated emission of electromagnetic radiation. Lasers differ from other sources of light because they emit light coherently. Its spatial coherence allows a laser to be focused to a tight spot, and this enables applications like laser cutting. (see https://en.wikipedia.org/wiki/Laser_light)

### soft tissue

In anatomy, the term soft tissue refers to tissues that connect, support, or surround other structures and organs of the body, not being bone. Soft tissue includes tendons, ligaments, gum or gingiva, mucosa, fascia, skin, fibrous tissues, fat, and synovial membranes (which are corrective tissue), and muscles, nerves and blood vessels (which are not connective tissue). (see https://en.wikipedia.org/wiki/Soft_tissue)

### soft-tissue laser surgery

In soft-tissue laser surgery, interaction of laser light with the soft tissue provides a special approach to surgery. A highly focused laser beam vaporizes the soft tissue. Laser can make very small incisions when the beam is focused on the tissue (focal spot size can be as small as = 0.1 mm, but the most widely used in practice is 0.4 mm). When the beam is defocused, the intensity of the laser light on the tissue diminishes, and it can be used for cauterization of small blood vessels and lymphatics, therefore decreases post-operative swellings. (see https://en.wikipedia.org/wiki/Soft-tissue_laser_surgery)

## Claims

1. A device (100) for treatment of the human or animal body by surgery with the help of laser light (102), in particular for dentistry, comprising:
- a laser diode with a wavelength of 445 ± 20 nm for providing the laser light;
- an electrical power supply (106) for the laser diode, wherein the power supplied to the laser diode is between 1 to 3 W;
- a conversion effectiveness of at least 50% of electrical to optical power leading to only 1,5W or less of thermal heating in the device;
- only a passive cooling to absorb the amount of thermal heating of the laser diode by the surrounding air and the hand of the operator.

2. A device as claimed in claim 1, the laser diode for providing the laser light having a wavelength of 445 ± 10 nm.

## Patentansprüche

1. Vorrichtung (100) zum Behandeln des menschlichen oder tierischen Körpers durch einen chirurgischen Eingriff unter Verwendung von Laserlicht (102), insbesondere in der Zahnheilkunde, die umfasst:
eine Laserdiode mit einer Wellenlänge von 445 ± 20 nm, um das Laserlicht bereitzustellen;
eine elektrische Leistungsversorgung (106) für die Laserdiode, wobei die der Laserdiode zugeführte Leistung zwischen 1 und 3 W beträgt;
eine Umwandlungseffektivität von elektrischer zu optischer Leistung von zumindest 50 %, die zu einer Wärmeerhitzung in der Vorrichtung von nur 1,5 W oder weniger führt;
nur eine passive Kühlung zum Absorbieren der Menge an Wärmeerhitzung der Laserdiode durch die Umgebungsluft und die Hand des Benutzers.

2. Vorrichtung nach Anspruch 1, wobei die Laserdiode zum Bereitstellen des Laserlichts eine Wellenlänge von 445 ± 10 nm aufweist.

## Revendications

1. Dispositif (100) de traitement d'un corps humain ou animal par chirurgie à l'aide de la lumière laser (102), en particulier, pour la dentisterie, comprenant
une diode laser avec une longueur d'onde de 445 ± 20 nm pour la fourniture de la lumière laser ;
une alimentation en courant électrique (106) pour la diode laser, où le courant alimenté dans la diode laser est entre 1 et 3 W ;
une efficacité de conversion d'au moins 50 % du courant électrique en énergie optique menant à seulement 1,5 W ou moins de réchauffement thermique dans le dispositif ;
uniquement un refroidissement passif pour absorber la quantité de réchauffement thermique de la diode laser par l'air environnant et la main de l'opérateur.

2. Dispositif selon la revendication 1, la diode laser diode pour fournir la lumière laser ayant une longueur d'onde de 445 ± 10 nm.
